(19) Europäisches Patentamt

European Patent Office

Office européen des brevets

(11) **EP 4 755 337 A2**

(12) **EUROPEAN PATENT APPLICATION**

(43) Date of publication:
**10.06.2026 Bulletin 2026/24**

(21) Application number: **26171152.7**

(22) Date of filing: **27.11.2024**

(51) International Patent Classification (IPC):
***A61B 34/10*** (2016.01)

(52) Cooperative Patent Classification (CPC):
**A61B 34/10;** A61B 34/25; A61B 2034/108;
A61B 2034/2055; A61B 2090/376; A61F 2002/4633

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(62) Document number(s) of the earlier application(s) in
accordance with Art. 76 EPC:
**24215694.1 / 4 751 674**

(71) Applicant: **Stryker European Operations Limited
Carrigtwohill, Co. Cork T45 HX08 (IE)**

(72) Inventor: **WEEDE, Oliver
79110 Freiburg (DE)**

(74) Representative: **Schott, Jakob Valentin
Wuesthoff & Wuesthoff
Patentanwälte und Rechtsanwalt PartG mbB
Schweigerstraße 2
81541 München (DE)**

Remarks:
This application was filed on 08.04.2026 as a
divisional application to the application mentioned
under INID code 62.

(54) **TECHNIQUE FOR AUTOMATIC SPINAL CAGE PLANNING**

(57) A technique for automatic spinal cage planning is provided. In particular, a computer-implemented method 200 for automatically planning implementation of one or more spinal cages is provided. The method 200 comprises obtaining 210 image data including a 2D medical image comprising representations of a plurality of vertebrae of a patient's body in a standing body posture and determining 220 a 2D prediction image comprising representations of the plurality of vertebrae of the patient's body in the standing body posture, wherein determining the 2D prediction image comprises modifying the 2D medical image such that at least one spinal balance parameter derivable from the 2D prediction image fulfills a predefined criterion. The method 200 further comprises determining 230, based on the 2D prediction image, an implementation of one or more spinal cages yielding the poses of the vertebrae as represented by the prediction image.

Fig. 2

EP 4 755 337 A2

## Description

### Technical Field

The present disclosure generally relates to the planning of spinal surgeries. In particular, a method for automatically planning an implementation of one or more spinal cages, a computer program product, and a planning system are presented.

### Background

Spinal fusion, also called spondylodesis or spondylosyndesis, is a surgery performed by orthopaedic surgeons or neurosurgeons that joins two or more vertebrae. Spinal fusion can be used to treat a variety of conditions affecting any level of the spine, e.g., lumbar, cervical and thoracic. In general, spinal fusion is performed to decompress and stabilize the spine.

There are many types of spinal fusion techniques. Each technique varies depending on the level of the spine and the location of the compressed spinal cord/nerves. After the spine is decompressed, bone graft and/or artificial bone substitute is packed between the vertebrae to help them heal together. In general, fusions are done either on the anterior (stomach), posterior (back), or both sides of the spine.

Most spinal fusion techniques use spine fusion instrumentation, e.g., screws, plates, rods, and/or cages because they have been shown to improve the union rates compared to non-instrumented fusion techniques. Based on the level of the spine and the location of the compressed spinal cord/nerves a variety of different instrumentation, e.g., differently sized and shaped cages may be used.

Which instrumentation is to be used and how to align the spine is commonly manually decided by a surgeon. Since the spine is a highly sensitive area with potentially fatal consequences in case the wrong instrumentation and/or alignment is used, manual planning of which spine fusion instrumentation to use is a very tedious task which can only be performed by an experienced surgeon and which puts a high cognitive load on the surgeon. The manual planning may be based on using a known software for simulating cages like "Surgimap", which allow the surgeon to simulate cages and manually adapt parameters thereof. However, even with such software multiple parameters have to be manually adapted which renders the task to determine an optimized cage extremely complex and tedious or even impossible.

Further, in addition to the manual planning, the suitability of the used instrumentation, e.g., a cage shape, may still have to be evaluated through trial fittings, with fluoroscopic imaging utilized to confirm the placement of the cage. Such trial fittings may result in additional risk for the patient, since suboptimal cages may be used and since the duration of a surgery may be increased. Further, such trial fittings are performed on a patient in a lying position so that a comparison to target alignment derived from a standing x-ray is only possible to a limited extent at best. In other words, a detailed determination of the spinal alignment based on fluoroscopic imaging of a patient in a lying position is impossible. Thus, such trial fittings are only suited to determine whether a cage fits in a disk space between adjacent vertebrae but not whether the target spinal alignment in the standing position will be achieved.

### Summary

There is a need for an improved planning technique addressing at least some of the aforementioned or other problems. In particular, there is need for providing a method for automatically planning an implementation of one or more spinal cages to improve at least one of the size, shape and positioning of the one or more spinal cages to be implanted. Using an automatic planning method may enable evidence based spinal surgery, e.g., to select a spinal cage and alignment that optimize spinal parameters, e.g., that result in spinal parameters that have already been proven to improve the results of a surgery, e.g., at least one of the mechanical stability of the cage(s) and the post operative well-being of a patient and thus result in less revisions. Further, using an automatic planning method may reduce the cognitive load on a surgeon. Summarized, the chances for a successful spinal fusion including post-operative well-being of a patient and mechanical stability of the implanted cage(s) may be increased due to determining an optimized spinal cage implementation, e.g., size, shape and/or positioning.

According to a first aspect, a computer-implemented method for automatically planning an implementation of one or more spinal cages is provided. The method comprises obtaining image data including a 2D medical image comprising representations of a plurality of vertebrae of a patient's body in a standing body posture and determining a 2D prediction image comprising representations of the plurality of vertebrae of the patient's body in the standing body posture, wherein determining the 2D prediction image comprises modifying the 2D medical image such that at least one spinal balance parameter derivable from the 2D prediction image fulfills a predefined criterion. The method further comprises determining, based on the 2D prediction image, an implementation of one or more spinal cages yielding the poses of the vertebrae as represented by the prediction image.

The obtained image data may be or comprise x-ray image data, e.g., a lateral, LAT, x-ray image. The image data may further comprise an anterior-posterior, AP, x-ray image. The obtained image data may be or comprise pre-operative image data allowing the planning to be performed pre-operatively. As a result, for example, the duration of the operation and thus the risk for the patient may be reduced. Additionally or alternatively, intra-operative image data may be obtained allowing for optimization of a planned implementation based on a current spinal shape indicated in the intra-operative image data.

In some variants, determining the implementation of the one or more spinal cages may comprise transferring the poses of the vertebrae as represented by the 2D prediction image onto vertebrae represented by a 3D patient image, and determining the implementation based on the vertebrae represented by the 3D patient image having the transferred poses. The 3D patient image may be or comprise a computed tomography, CT, scan of a patient.

The method may further comprise determining a registration for registering the 2D medical image with the 3D patient image, wherein the poses of the vertebrae as represented by the 2D prediction image are transferred onto vertebrae represented by the 3D patient image based on the registration of the 2D medical image with the 3D patient image. The registration may be determined based on known registration techniques, e.g., a point to point registration. The registration or landmarks for the registration may be automatically determined using a neural network, e.g., a deep neural network.

The transferred poses of vertebrae of the 3D patient image may be further adjusted, in one or more planes differing from an imaging plane of the 2D medical image, before determining the implementation of the one or more spinal cages based on the vertebrae represented by the 3D patient image that have the so-adjusted poses. The further adjustment of the transferred poses may comprise at least one of a cobb angle alignment and an axial alignment of the vertebrae. In some variants, the cobb angle and the axial angle have optimal values (e.g., of zero degrees) and the transferred poses may be determined to be as close as possible to the optimal values.

In some variants, determining the 2D prediction image may comprise simulating a fusion of different vertebra levels, e.g., an L5-L4 fusion and/or an L5-L3 fusion. A prediction image may be determined for each simulated fusion. The fusion of different vertebra levels may comprise a fusion of at least three vertebra levels, e.g., S1-L4, L5-L3, L4-L2, L4-L1 or L3-L1. Additionally or alternatively, the fusion may be simulated based on a predefined maximum number of vertebra levels to be fused.

The method may further comprise (e.g., when at least three vertebra levels are to be fused) outputting a respective optimal fusion result for each number of vertebra levels to be fused, e.g., for three levels, for four levels, for five levels. The method may comprise receiving user-input selecting one of the respective optimal fusion results, and determining the implementation of the one or more spinal cages based on the selected respective optimal fusion result.

Alternatively to the user-input (also referred to as manual input herein) for the selection of the levels to be fused, the fusion levels may be selected automatically. For example, simulating a fusion of different vertebra levels may comprise evaluating all 2-level fusions, followed by the assessment of higher-level fusions. The method may comprise evaluating the results with respect to a predefined threshold indicative of an improvement of the at least one spinal balance parameter resulting from adding another level to the fusion. If the improvement falls below the predefined threshold, the optimal outcome from the fusion with fewer levels may be selected. This systematic approach allows for comprehensive exploration of fusion scenarios and facilitates the identification of the most suitable wedge configuration to optimize spinal alignment and biomechanical stability.

As fusing more levels means a more invasive procedure and restricts mobility of the patient further, determining an appropriate threshold deciding whether it is beneficial to extend the surgery by one additional level may be useful. The method may thus comprise presenting a result of a chosen threshold to a user as a suggestion, said threshold limiting the number of levels to be fused. The suggestion may be adapted based on obtained user input in response to the presentation. As a result, a user, e.g., a surgeon may further specify the number of levels and constrain the automatically determined suggested optimization.

In some variants, simulating the fusion of different vertebra levels may comprise introducing at least one virtual wedge between vertebrae that are simulated to be fused together, for example between vertebrae represented by the 2D medical image or vertebrae represented by a or the 3D patient image. Introducing a wedge may comprise splitting the obtained 2D medical image into at least two parts and moving the at least two parts relative to each other, optionally within 3 degrees of freedom, e.g., two translational degrees of freedom and one rotational degree of freedom. The degrees of freedom may simulate an anterior-posterior translation, a cranial-caudal translation and a lordosis or kyphosis amendment. Each wedge may simulate the shape, size and pose (e.g., at least one of a position and an orientation) of a spinal cage to be implanted. Additionally or alternatively to the relative movement, simulating the fusion of different vertebra levels may comprise moving the 2D prediction image, e.g., rotating the prediction image to simulate a pelvic tilt compensation.

In some variants, the at least one spinal balance parameter may be indicative of at a global alignment and proportion, GAP, score. For example, the at least one spinal balance parameter may be indicative of at least one of a pelvic tilt, PT, a thoracic compensation, a knee flexion, a disc height restauration, an anterior-posterior alignment, a sacral slope, SS, a lumbar lordosis, LL, lumbar lordosis distribution, LDI, a global tilt, GT, a T1 pelvic angle, TPA, a L4-S1 lordosis, a L1 pelvic angle, L1 PA, and a mismatch between a T4 and an L1 pelvic angle, T4PA-L1PA. The at least one spinal balance parameter may be derived from the 2D prediction image. Further spinal balance parameters may include at least one of a sagittal vertical axis, SVA, a T1 spinopelvic inclination, T1 SPI, and a T9 spinopelvic inclination, T9 SPI. Deriving the at least one balance parameter may comprise measuring a value of one of the aforementioned, e.g., a pelvic tilt, in the 2D prediction image and comparing the measured value to an optimal value, e.g., by determining a relative conformity or deviation between the measured value and the optimal value. In other words, a derived spinal balance parameter may be indicative of a relation between a measured value and an optimal value and thus indicative of the quality of the measured value.

In some variants, the method may further comprise obtaining one or more patient specific parameters and deriving the at least one spinal balance parameter based at least in part on the one or more patient specific parameters. The one or more patient specific parameters may be or comprise at least one of a parameter derived from the obtained image data, e.g., based on measuring a pelvic incidence indicated by the image data, metadata associated with the image data or independently provided data. The one or more patient specific parameter may comprise at least one of gender, age and body mass index. Based on the patient specific parameters, upper and lower constraints for values associated with the spinal balance parameters mentioned herein may be defined, e.g., to limit the algorithms for determining the implementation to practically realistic values.

In some variants, multiple spinal balance parameters may be derived from the 2D prediction image and the implementation of one or more spinal cages may be determined based on a combination of at least some of the multiple spinal balance parameters. Combining the at least some of the multiple spinal balance parameters may comprise determining a weighted sum of the derived multiple spinal balance parameters. The method may further comprise determining, based on the weighted sum, a deviation, D(x), of the poses of the vertebrae as represented by the 2D prediction image from an optimal value for the poses of the vertebrae. The deviation may be defined as:

$$D(x) = 1 - \Sigma\, w_i * p_i\,(x);\ i = 1, ..., n; \qquad\qquad (1)$$

with D as the deviation, x as a measured value, $w_i$ as a weight and $p_i$ as a spinal balance parameter. According to the definition of equation (1), $p_i(x)$ defines the conformity of a measured value to an optimal value. The above definition for the deviation allows for a fast and easy determination of the quality of the position of the vertebrae as represented by the 2D prediction image and also the quality of specific values allowing a straightforward optimization problem, which may be solved automatically. For example, a deviation of zero, e.g., $p_i = 1$ for i = 1 to n (at least when the weight $w_i$ is not zero), is indicative for an ideal condition. In some examples, a parameter $p_i$ between 1 and 2/3 may be identified as aligned, e.g., as fulfilling the predetermined criterion, a parameter between 2/3 and 1/3 may be identified as moderately misaligned and a parameter between 1/3 and 0 may be identified as severely misaligned, e.g., as not fulfilling the predetermined criterion. Different alignment interpretations, e.g., different predetermined criteria, are contemplated. For example, the interpretation may comprise a finer or coarser differentiation of alignment states and/or the differentiation between the alignment states may be non-symmetric. In some variants the predetermined criterion may only be fulfilled when the deviation according to equation (1) and/or the deviation between a dedicated measured value and an associated optimal value is smaller than 20%, smaller than 10% or smaller than 5%. Other known mathematical methods for evaluating the quality of the position of the vertebrae as represented by the 2D prediction image are contemplated.

In some variants, deriving the multiple spinal balance parameters from the 2D prediction image may comprise determining multiple spinal balance parameters sets and selecting one of the sets for determining the implementation based on at least one optimization criterium.

In some variants, determining the implementation of the one or more spinal cages may comprise at least one of determining a position and orientation of the one or more spinal cages and determining a respective shape for each of the one or more spinal cages based at least in part on the 2D prediction image or the 3D patient image comprising the transferred poses. In other words, the position, orientation and shape of each of the one or more spinal cages may be determined so that a spine of the patient, after an implementation of the one or more spinal cages, corresponds to the spine indicated in the 2D prediction image or the 3D patient image comprising the transferred poses, e.g., based on the virtual wedges introduced in the images that result in an optimized deviation as explained above.

In some variants, the method may further comprise selecting, based on the determined respective shapes, a spinal cage from a family of predetermined spinal cages matching the determined respective shape the best. Additionally or alternatively, the method may comprise outputting instructions for manufacturing a spinal cage that matches one of the determined respective shapes.

In some variants, determining the implementation of the one or more spinal cages may comprise determining, based at least in part on the 2D prediction image or the 3D patient image comprising the transferred poses, a region within a space between two adjusted vertebrae to place one of the one or more spinal cages in, and determining a position for said

spinal cage within the determined region based at least in part on the 2D prediction image. This may be done for each of the one or more spinal cages, for example such that each region is virtually filled by a respective spinal cage.

In some variants, the method may further comprise triggering display of a visualization indicating the determined spinal cage implementation together with the selected spinal cage. The visualization of the selected spinal cage may comprise a visualization of one or more dimensions of the cage, like length, height, width and lordotic angle, and/or a serial number of the cage. The visualization may further comprise indicating the optimal fusion levels of the plurality of vertebrae, e.g., $S_i$-$L_j$ or $L_i$-$L_j$.

In some variants, the visualization may comprise at least one of a visualization in the 2D prediction image, a visualization in the 3D patient image comprising the transferred poses, and a visualization of the selected cage attached to a cage insertion instrument. The visualization described herein may be triggered to be displayed on one or more displays.

In some variants, the method may further comprise receiving user-input adjusting at least one of the visualized spinal cage implementation and the selected spinal cage, and re-calculating the spinal cage implementation and the spinal cage shape based on the user-input. The user-input may indicate specific levels of vertebrae to be fused or a specific number of vertebra levels to be fused. The received user input may adjust specific parameters $p_i$ and/or the weights $w_i$ thereof.

According to a second aspect, a computer program product is provided, comprising instructions that, when executed on at least one processor, cause the at least one processor to carry out the method described herein. In particular, the instructions, when executed on the at least one processor, cause the at least one processor to carry out any of the variants of the method according to the first aspect.

According to a third aspect a planning system is provided comprising at least one processor configured to carry out the method described herein. In particular, the at least one processor is configured to carry out any of the variants of the method according to the first aspect.

In some variants, the planning system may further comprise at least one of the following entities: a medical imaging device configured to generate at last one of the 2D medical image and the 3D patient image, a user input device configured to receive the user-input, a display device configured to display the visualization.

## Brief Description of the Drawings

Further details, advantages and aspects of the present disclosure will become apparent from the following embodiments taken in conjunction with the drawings, wherein:

Fig. 1       shows a planning system for automatically planning an implementation of one or more spinal cages;

Fig. 2       shows a flow diagram of a method for automatically planning an implementation of one or more spinal cages;

Fig. 3A      shows a schematic example of 2D image data indicating a spine of a patient before the implementation planning;

Fig. 3B      shows a schematic example of a 2D prediction image;

Fig. 4A      shows an image of a suggested spine cage implementation with an indication of different spinal balance parameters as well as a user interface for adjusting the suggested spinal cage implementation; and

Fig. 4B      shows a 3D prediction image and a 2D prediction image indicating a planned spinal cage implementation and spinal balance parameters.

## Detailed Description

In the following description of exemplary embodiments, the same reference numerals are used to denote the same or similar components.

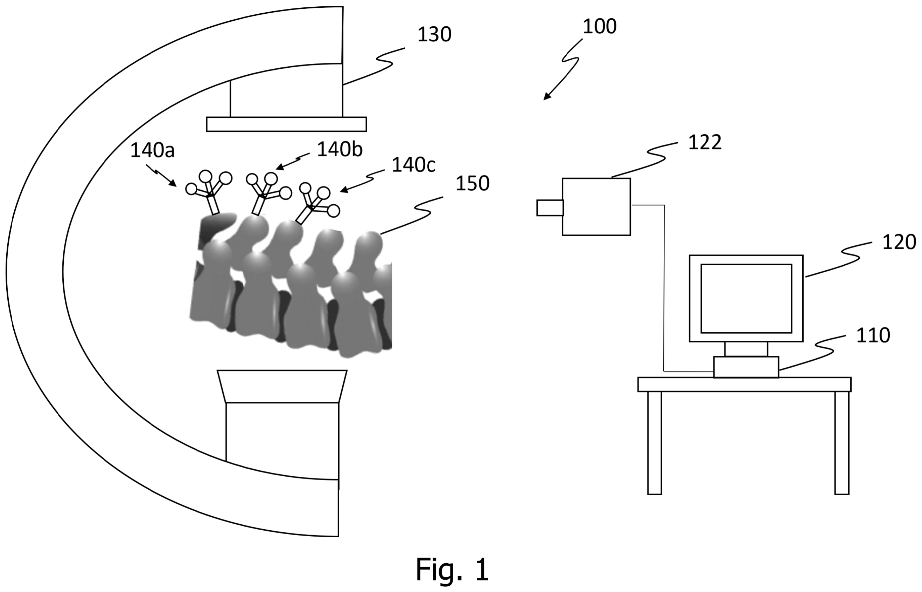

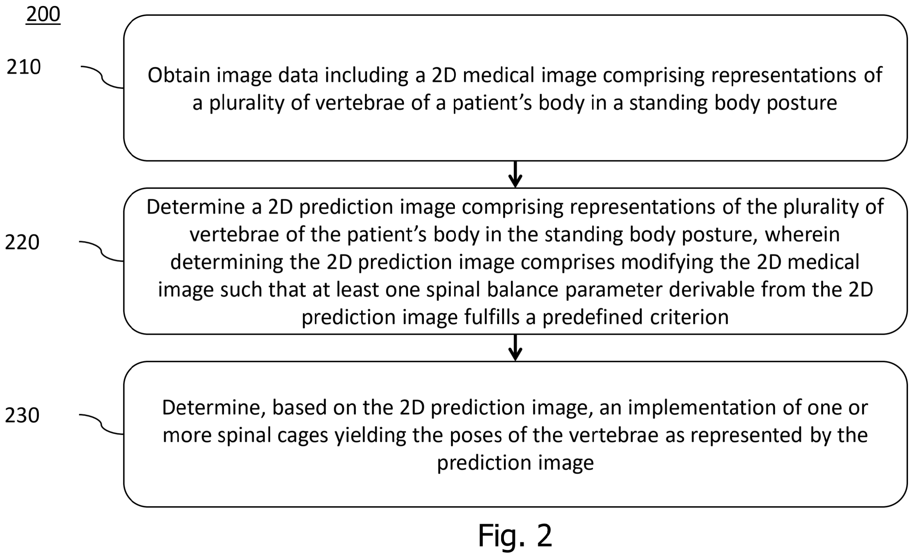

Fig. 1 shows a planning system 100 for automatically planning implementation of one or more spinal cages. The planning system 100 comprises a computing device 110 having a processor configured to carry out the method as explained in detail with reference to Figs. 2 to 4B below. The computing device 110 may be any known computing device 110 comprising one or more processors, e.g., a personal computer, or a server.

The system 100 further comprises a display device 120 for displaying data associated with the planning. In some variants, the display device 120 may be or comprise a touch display configured for receiving user input. Additionally, or alternatively, the planning system 100 may comprise a dedicated user input device (not shown in Fig. 1), e.g., a computer mouse and/or keyboard. Moreover, the system 100 comprises a tracking camera 122 configured for generating tracking data that may be used for intra-operative registration and/or navigation purposes. For example, a planned spinal cage may be visualized within medical image data during implantation of the planned spinal cage based on a registration determined based on the tracking data. The visualization of the planned spinal cage may be used for at least one of navigation purposes, determining whether the planned alignment is achieved in practice, and determining an adapted position for achieving the planned alignment.

Still further, the system 100 comprises a medical imaging device 130 for generating 2D and/or 3D medical image data, e.g., x-ray images or CT scans. The shown medical imaging device 130 comprises a common C-arm. Other known imaging devices, e.g., devices comprising an o-arm, are contemplated.

Moreover, the system 100 comprises multiple optical trackers 140a, 140b 140c trackable via the tracking camera 122. Each of the trackers 140a, 140b 140c is attached to a different vertebra allowing the tracking camera 122 to generate the tracking data for the tracking/navigation/further determination purposes explained above.

Fig. 2 shows a flow diagram of a method 200 for automatically planning an implementation of one or more spinal cages. The method 200 comprises a first step 210 of obtaining image data including a 2D medical image comprising representations of a plurality of vertebrae of a patient's body in a standing body posture.

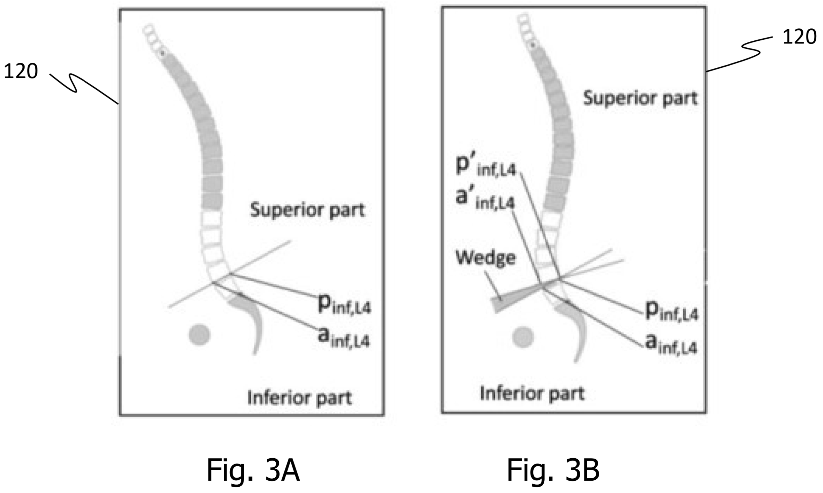

Fig. 3A shows a schematic example for a 2D medical image that can be displayed on the display 120 of the system 100. In the shown example, the spine is separated into a superior part and an inferior part as indicated by the line drawn at the bottom of the vertebra L4, e.g., between the vertebrae L4 and L5, wherein a position $p_{inf,L4}$ of a lower posterior edge and a position $a_{inf,L4}$ of a lower anterior edge of the vertebra L4 are indicated for illustrative purposes. Such a LAT 2D medical X-ray image may be obtained in step 210, the division into the superior and inferior part may be automatically determined based on a segmentation of this image.

Returning to Fig. 2, the method 200 comprises a second step 220 of determining a 2D prediction image comprising representations of the plurality of vertebrae of the patient's body in the standing body posture. Determining the 2D prediction image comprises modifying the 2D medical image obtained in step 210 such that at least one spinal balance parameter derivable from the 2D prediction image fulfills a predefined criterion.

A schematic example of a 2D prediction image displayed on the display 120 of the system 100 is shown in Fig. 3B. In this example, the superior part of the spine as defined in Fig. 3A has been rotated relative to the inferior part as defined in Fig. 3A. In detail, the superior part has been rotated around the indicated posterior point $p_{inf,L4}$. As a result, a gap in the form of a wedge has been introduced between the vertebrae L4 and L5 as indicated in Fig. 3B. The form of the wedge may be determined based on a spatial relation between the positions $p_{inf,L4}$ and $a_{inf,L4}$ of the indicated edges prior to the rotation and positions $p'_{inf,L4}$ and $a'_{inf,L4}$ after the rotation. While in the shown example only the single rotation as explained above has been performed for clarity purposes, the modification of the 2D medical image may comprise a rotation and one or more translations between two adjacent vertebrae, e.g., between a vertebra pair. Further, respective rotations and translations may be performed between respective vertebra pairs, e.g., between two or three or more respective vertebra pairs, so that not only a superior and inferior part of the spine are defined but multiple parts. The respective rotations and translations may be different for each respective vertebrae pair resulting in respective wedges between the vertebrae pairs.

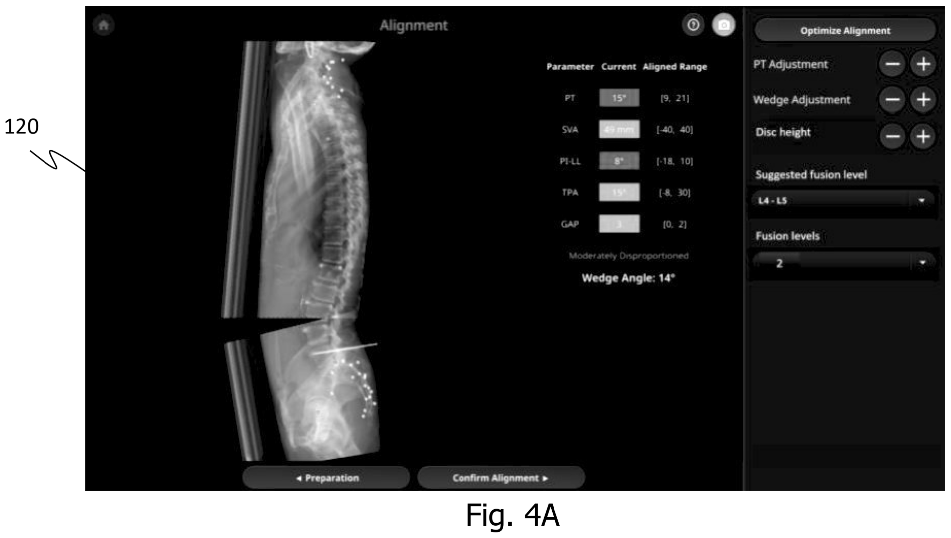

Based on the 2D prediction image, balance parameters $p_i$ may be derived as described herein, e.g., with reference to equation (1). Fig. 4A shows a set of derived balance parameters displayed next to a 2D prediction image. In detail, the names of the parameters of the parameter set are shown next to measured values associated with respective parameters of the parameter set. Each measured value is displayed within a window having a colored background indicating whether the parameter fulfills the predefined criterion, e.g., based on a comparison between the shown measured value and a predefined optimal value associated with the respective balance parameter. For example, a green background indicates that the predefined criterion is fulfilled while an orange and/or a red background indicate that the predefined criterion is not-fulfilled. The relative difference between the shown measured value and an optimal value may also be indicated by the colored background, e.g., with green indicating a small relative difference, e.g., smaller than 33 % orange indicating a medium relative difference, e.g., between 33 % and 66 % and red indicating a great difference, e.g., greater 66 %. The indications may comprise more or less or different colors and may be based on different ranges than the ones of the previous example. The optimal values may be defined based on the GAP score.

Further an upper and a lower threshold for the measured values are shown in Fig. 4A. These thresholds are predefined constraints to exclude theoretically possible but practically non-possible values from the automated determination steps to optimize the implementation planning.

Further, Fig. 4A shows a section of the display 120 configured as a touch display for receiving user input. The user input may define an adjustment of the prediction image. The shown adjustment options comprise a PT adjustment, a wedge adjustment (e.g. an adjustment of the opening angle of the wedge) and a disc height adjustment (e.g., an adjustment of the height and position of the wedge). The aforementioned adjustments may result in an adjusted rotation and/or adjusted translations between two neighboring vertebrae. Further the user input may define the number of spine levels that are to be fused and, still further, may explicitly define which spine levels are to be fused. After receiving user input, a re-planning based on the adjusted parameters may be triggered.

Returning again to Fig. 2, the method 200 further comprises a third step 230 of determining, based on the 2D prediction image, an implementation, e.g., the form position and orientation, of one or more spinal cages yielding the poses of the vertebrae as represented by the prediction image. For example, a region between two vertebrae in which a spinal cage may be optimally located may be determined based on a wedge as shown in Fig. 3B. Determining the region may comprise limiting the wedge based on predefined boundaries, e.g., based on a planned surgical approach and/or to prevent a protrusion of a spinal cage between the vertebrae, when the spinal cage is in the optimal position. Based on the determined region, a form, position and orientation of a spinal cage may be determined. The implementation of one or more spinal cages may further be determined based on additional information determinable from a 3D prediction image, e.g., a length of a disc space between two adjacent vertebrae. For example, the implementation for a lateral spinal cage may be determined based on a length of a disc space along a transversal axis thereof. The form of the spinal cage may comprise a lordotic angle, a cage height, a cage length and a cage width.

While the examples shown in Figs. 3B and 4A above are directed at a 2D prediction image, the poses of the vertebrae in the 2D prediction image may be transferred to 3D image data and the determinations explained with reference to the 2D image data could analogously be performed based on the 3D image data having the transferred poses.

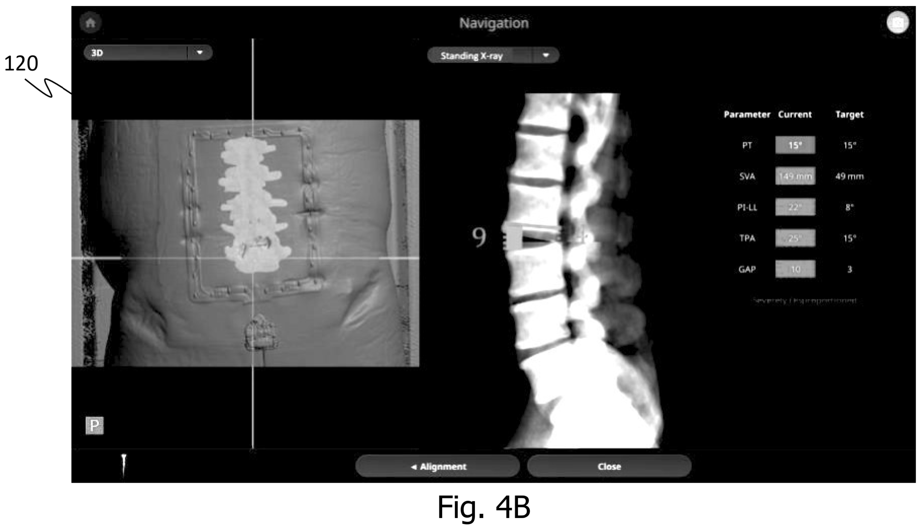

Further, Fig. 4B shows a 3D prediction image and a 2D prediction image indicating a current set of spinal balance parameters analogously to Fig. 4A and an associated set of target spinal balance parameters to be achieved when the indicated cage is implemented according the planning. Additionally, Fig. 4B shows the target position of the superior vertebra indicated by its inferior endplate. As a result, a user may easily derive from the shown values and the indicated target position whether a correct implant is implanted as planned.

In some examples, the method may comprise obtaining intra-operative image data and displaying the planned spinal cage implementation together with the intra-operative image data.

In some examples, tracking data indicative of poses of tracked vertebrae and/or a tracked instrument for inserting a spinal cage may be obtained and the intra-operative image may be adapted based on the tracking data allowing a real-time comparison between the current spinal balance parameters and the spinal balance parameters determined in the planning.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

The present technique will now be explained in other words. It is to be understood that the features disclosed in the following may be combined with the features discussed herein above and vice versa.

One objective of spinal fusion surgery is to fuse operative levels in a manner that restores a patient's balance, including alignment of the spinal curvature and restoration of disc height. Therefore, spinal fusion surgery commonly involves the insertion of interbody devices (cages), into the disc space. In conventional practice, alignment (e.g., part of an implementation) planning is frequently omitted, leaving the task solely to the surgeon's expertise. The suitability of, e.g., a shape/size (e.g., in combination with a position and orientation) of a spinal cage is commonly evaluated through trial fittings with (e.g., intra-operative) fluoroscopic imaging utilized to confirm a placement (e.g., as part of an implementation) of the spinal cage (e.g., based on intra-operative fluoroscopy images). Alternatively, existing software facilitating manual planning may be used by a surgeon. Omitting of planning may increase a health risk for a patient or decrease the mechanical stability of a spinal cage. Further, manual planning may be prone to human errors and thus put a high cognitive load on a surgeon and may only be performable by highly experienced surgeons.

Thus, there is need for an improved technique for planning of an implementation of one or more spinal cages.

In some variants of the third aspect explained herein, an automatic spinal alignment and interbody device (spinal cage) planning system 100 for spinal fusion surgery may be provided, for example, as extension of a navigation system. The system 100 may be configured for optimizing spinal curvature and balance in an upright position (e.g., according to steps 210 and 220 as described herein with reference to Fig. 2 / based on x-ray images in the standing position and respective optimal values for one or more spinal balance parameters). Moreover, the system 100 may be configured to provide a cage planning (e.g., according to step 230 as described herein with reference to Fig. 2 / implementation, e.g., shape, position and orientation planning of one or more spinal cages) supporting the computed, e.g., optimized spinal curvature and balance in the upright position. In some variants, the planning system 100 may additionally be configured for providing intra-operative feedback regarding the achievement of alignment goals and spinal cage placement.

A determined plan (e.g., for an implementation of a spinal cage) may be automatically generated and visualized after loading patient data allowing surgeons to modify and customize it to suit various surgical procedures. Furthermore, in cases where the pre-operative plan cannot be achieved, intra-operative re-optimization of interbody planning (e.g., re-calculating of an implementation plan based on obtained intra-operative image data) enables the calculation of the ideal interbody device (e.g., spinal cage) shape and pose to best accommodate the current spinal shape. The present technique may thus allow automatic planning within a navigation system directly after intra-operatively scanning the patient. With this capability the technique may provide a step towards evidenced based spine surgery and allows surgeons to optimize spinal parameters "on-the-fly".

For interbody (e.g., spinal cage implementation) planning, a 3D model may be used, whereas a 2D model derived from the LAT standing x-ray (e.g., a Lateral Prediction Image) may be sufficient to model aspects for lateral alignment. For lateral alignment automatic cage planning may (e.g., in some variants according to the first aspect described herein) be divided into: (a) Pre-operative calculation of an alignment and balancing target based on a patient's standing lateral full-spine x-ray (e.g., based on a lateral prediction image, for example according to steps 210 and 220 described herein) (b) Calculation of a 3D cage position and shape based on this target in a 3D prediction image, created by reformatting the operative levels of 3D imaging data such as a patient's computed tomography scan used for navigation.

The technique is not limited to this separation. For example, an anteroposterior full spine x-ray (e.g., scoliosis view) may be added for coronal adjustment; or the optimization may be performed (e.g., directly) on an 3D model that represents the standing patient condition (e.g., based on 3D patient images having adjusted poses as described herein).

For evaluating the balance and alignment of the spine, several parameters from medical literature may be considered.

- Spinal Alignment and Balancing Parameters, which are important for the health of the patient and the mechanical stability of the construct (e.g., vertebrae). Alignment targets describe the patient specific ideal shape of the vertebrae, whereas balance targets focus on Duboussets "cone of economy", to ensure that the head is located over the pelvis and to give the patient the least amount of energy expenditure to maintain an upright posture (e.g., SVA close to zero). Balance parameters are dependent on the alignment of the spine and on compensation mechanisms of the patient, like the Pelvic Tilt, Thoracic compensation, or knee flexion. Whereas alignment targets such as the lumbar lordosis (LL) are independent of these mechanisms and describe (e.g., local) alignment of the vertebrae in an asymptomatic individual.

- Disc Height: Restoring the optimal disc height is crucial for decompression and the stability as it decreases distractive and compressive forces on endplates.

- Anteroposterior (AP) Alignment: In addition, achieving alignment of vertebral bodies along the anterior-posterior axis is crucial for decompression and ensuring spinal stability during spinal fusion procedures.

Some variants according to the first aspect may comprise at least some of the following steps:

- obtaining a medical image, e.g., a 3D navigation image / 3D medical image data;
- obtaining a lateral, LAT, standing x-ray image (e.g., a 2D x-ray image);
- determining, based on image data, a 3D prediction image with optimized alignment, e.g., by:

   o registering the 2D and 3D image data;
   o creating a sagittal alignment target by optimizing an objective function, wherein the objective function may be iteratively optimized, and, e.g., by:

      ▪ creating a lateral prediction image (e.g., 2D prediction image) by modifying the standing x-ray image, e.g., by:

         • dividing the standing x-ray into image portions and changing a pose of the image portions;
         • applying a rotation (e.g., around a femur axis to simulate a pelvic tilt) to the modified medical image, e.g., to the x-ray comprising the changed poses; and
         • deriving patient specific parameters from the prediction images;

      ▪ computing the objective function based on patient specific information, e.g., like a T1 pelvic angle and/or a pelvic tilt;

   o creating a 3D prediction image / modifying the 3D navigation image, e.g., by:

      ▪ aligning coronal and axial aspects;

- computing spinal cage positions (e.g., a spinal cage implementation comprising at least one of a position, an orientation, and a shape of a spinal cage) in a prediction image, e.g., by:

   o obtaining alignment data;

○ measuring disk space;
○ optimizing a cage position and shape; and
○ obtaining implant geometries of a given implant system (e.g., of a predetermined family of spinal cages)

- visualizing (e.g., of the computed implementation) and receiving optional user input; and
- receiving intra-operational image data and/or tracking data (e.g., indicative of at least one tracker associated with, e.g., one or more vertebrae or a tool for inserting a spinal cage) for determining movements (e.g., of one or more vertebrae and/or the tool) and triggering display thereof.

In the steps described above, some of the following data may be obtained and used:

- LAT Standing x-ray for creating the lateral prediction image;
- 3D Navigation Image for registration of the standing x-ray and optionally for deriving movement constraints. It may be used for creating the 3D prediction image on which cage planning may be performed;
- anatomical information for spinal parameters computation, depended on the chosen parameter set to be optimized, e.g.:

    o vertebral levels included in the fusion (e.g., L5-L4);
    o position of vertebral landmarks, at least the inferior and superior vertebrae of a fusion;

        ▪ e.g. superior/inferior anterior/posterior corners of the endplates;
        ▪ landmarks for spinal parameter computation, such as the center of C7 for SVA calculation;

    o registration information for the standing X-ray such as corresponding endplate points in the 3D image and the standing x-ray;
    o femur head positions for pelvic tilt simulation;
    o a (e.g., full) vertebra segmentation in the 3D image to capture the exact surface and to perform geometrical computations regarding dexterity and collisions; furthermore, a bio-mechanical model may be used to enable highly accurate simulation of movement constraints and forces;

- Geometry information (e.g., the shape / width, length, height, lordotic angle) of cages to determine the ideal cage that fits into the optimized location (the ideal cage may be referred to as a "3D implant hull");
- Tracking data indicative of multiple vertebrae poses to enable intra-operative optimization of cage planning.

In more detail, the 3D (medical) imaging data such as a patient's computed tomography scan may be obtained by the system 100. For example, the obtained 3D imaging data may be or comprise a 3D image based on which a navigated spine surgery is performed. Depending on the surgical approach the 3D imaging data may capture the operative levels of the patient in a position that differs from the standing position, e.g. the patient being in the prone or lateral position.
Vertebrae may be segmented automatically, and landmarks may be identified for registering a standing X-ray.
In addition to the 3D imaging data, a standing X-ray may be loaded into the system 100 (e.g., obtained by the system 100).
Landmarks for registration and spinal parameter calculation including endplate positions may be identified.
Segmentation and/or landmark identification in 2D and 3D imaging data may be derived automatically with a Deep Learning network.
An optimal vertebral repositioning may be simulated to obtain a post-operative prediction image estimating the spinal shape in the standing posture.
After calculation of the alignment goal, the exact size and position of the interbody device may be computed in a 3D prediction image.
The mapping between the, e.g., 3D navigation image and the LAT standing x-ray image may be derived by a 3D to 2D registration process. Establishing the mapping may allow length measurements in the standing x-ray without additional reference objects. The mapping may be needed for transferring a computed cage position from the lateral image to the 3D image and for transferring measurements of vertebral positions in a navigation system to the standing x-ray. The latter may be needed for intra-operative adjustment of a cage plan in cases where the pre-op plan could not be achieved.
There are several well-known methods known for 3D to 2D registration. A simple point-to-point registration may be applied under the assumption that the lateral standing x-ray is perfectly lateral oriented. Anterior and posterior endplate points may be chosen as landmarks for establishing the point-to-point registration. Determining the registration may comprise projecting landmarks of the 3D navigation image to the sagittal plane and deriving a 2D registration matrix that transforms corresponding points from the sagittal projection to the standing lateral x-ray with a known landmark transformation algorithm. As landmarks, a pair of anterior and posterior points of the superior endplate of the inferior reference vertebrae may be used.

After creating an aligned 2D prediction image, resulting positions may be transferred back to the 3D navigation image by applying the inverse transformation. By incorporating the initial transformation in the lateral image into the transformation chain, also intra-operatively measured 3D positions of vertebrae may be reformatted to the standing x-ray. This allows estimation of a standing x-ray in an upright position of the patient.

Calculate the sagittal alignment and balancing target may include compensation mechanisms like Pelvic Tilt, disc height restauration and anterior-posterior alignment by optimizing the objective function. The optimization may be described as:

$$p_{optimal} = argmin_\theta\ f(p) \hspace{3cm} (2)$$

with p describing the position of the vertebrae in the lateral prediction image, θ describing the movement constraints of the vertebrae, and f being an objective function that is minimized to get an optimal alignment $p_{optimal}$. The optimal alignment may describe the position of the vertebrae of the aligned and balanced spine in the lateral prediction image including compensation mechanisms.

To optimize the objective function, iterative metaheuristics for global optimization may be used such as multi-level gradient decent. Hence, various simulated positions of the vertebrae that meet specified movement constraints, may be evaluated by the objective function, and the best solution may be chosen. Thus, after several iterations the alignment may be approximated that optimizes the objective function.

As lateral alignment and balancing parameters may need to be measured in the upright standing position, potential vertebral movements of the instrumented levels may be simulated by modifying the standing x-ray image to calculate a lateral (2D) prediction image (as shown in Fig. 3B).

Figs. 3A and 3B show a potential modification of a standing X-ray by introducing a wedge as a result of a transformation of the superior image (part) in respect to a modified vertebral level (e.g. L5-L4). Therefore, the (2D prediction) image may be divided into superior and inferior parts, both may be treated as rigid.

To achieve the optimal alignment the simulation (the computer-implemented method according to the first aspect as described herein) may comprise evaluating a fusion of different levels, such as a L5-L4 fusion, or an L5-L3 fusion. Each time, a prediction image may be created by introducing at least one wedge.

Dependent on the use case, the simulation may be performed just for a fixed level such as L5-L4, for any adjacent pair of levels, or for multi-level fusions:

• Fixed 2-level fusion

    o This option involves a predefined fusion of two vertebrae, such as L5-L4, to determine one optimal wedge between the specified vertebrae.

• Variable 2-level fusion

    ○ In this variant, the simulation is applied to all adjacent vertebrae in a given range (e.g., S1 to L1), resulting in one optimal wedge between any of the adjacent levels (S1-L5, L5-L4, L4-L3, L3-L2 or L2-L1).

• Variable multi-level fusion

    o E.g. all 3-level fusions of adjacent vertebrae starting with S1 as the inferior vertebrae and ending with L1 as superior vertebrae, namely the S1-L4, L5-L3, L4-L2 and L3-L1 fusion.

The selection of instrumented levels may be determined automatically based on the simulation of the different (level fusion) approaches and the resulting values of the objective function. In detail, the simulation process may start by evaluating all 2-level fusions, followed by the assessment of higher-level fusions. Generally, a fusion of more levels tends to minimize the gap between the ideal and simulated values. If the improvement resulting from adding another level falls below a predefined threshold, the optimal outcome from the fusion with fewer levels my be selected.

This systematic approach may allow for comprehensive exploration of fusion scenarios and facilitates the identification of the most suitable wedge configuration to optimize spinal alignment and biomechanical stability.

As fusing more levels, may mean a more invasive procedure and may restrict mobility of the patient, determining an appropriate threshold deciding whether it is beneficial to extend the surgery by one level may be critical. Thus, the result of a chosen threshold may be presented (visualized / displayed) as suggestion to a user, e.g., a surgeon, but allowing the surgeon to specify the number of levels and constrain the optimization accordingly.

For deriving a lateral (2D) prediction image, an obtained (2D medical) image may be divided into image portions which are transformed, e.g., by:

- dividing the image into image portions, e.g., by splitting the image at the level, where the fusion is simulated, for example by choose cutting line through the anterior and posterior endplate points of the inferior endplate of the superior vertebrae; and
- transforming superior part of the image, e.g., by choosing a reference point in the superior image, e.g. the posterior, inferior endplate point of the superior vertebra, translating the superior part of the image including the reference point, and rotating the superior part of the image around the resulting reference point.

The transformation applied to the superior image part may consist of 3 degrees of freedom: AP-translation, cranial-caudal translation (disc height restauration) and rotation (lordosis/kyphosis increase).

For each fusion level a respective transformation may be computed and applied to the superior part of the standing x-ray (2D medical image), to create a lateral (2D) prediction image, creating a so called "wedge".

For simulating a pelvic tilt, the whole prediction image may be rotated around the femur head. If the lateral image is not perfectly sagittal two femur heads might be visible and the center of both may be chosen as rotation point. Other compensations mechanisms may be included to model wedges in the thoracic region.

Patient specific parameters may be obtained, which may be used or even necessary for computing spinal parameters and evaluating their values, as follows:

- measured in the imaging data (e.g. pelvic incidence),
- derived from the DICOM data (e.g. gender, age), and
- optionally provided to the system 100 (e.g. BMI).

After creating a lateral prediction image to be evaluated, the objective function may be computed based on measurements in the prediction image. The objective function may quantify the deviation from ideal values across a defined set of parameters to provide a comprehensive assessment of spinal health and function, guiding clinical decision-making and therapeutic interventions aimed at optimizing spinal alignment and biomechanical stability.

The objective function may be calculated as a weighted sum of parameters $p_i$ as defined in equation (1) above. The objective function may depend on the position of the vertebrae, e.g., based on the vertebra position, the optimal values may change.

Examples for ideal values derived from the GAP score are:

- 

$$SS = PI \cdot 0.59 + 9$$

- 

$$LL = PI \cdot 0.62 + 29$$

- 

$$LDI = L4S1Lordosis / L1S1Lordosis \cdot 100\,\% = 65\,\%$$

- 

$$GT = 0.48 \cdot PI - 15$$

Additional aspects focusing on upper parts of a spine may be included as well as thoracic compensation mechanisms.

For each of the parameters $p_i$ an ideal value may be provided, a normative range, a range of moderately misaligned state and a severely misaligned state. Constraints may be included for the optimization to capture potentially possible values, and to for example exclude a Pelvic Tilts outside the range of -5 to 40°. Additional constraints may be derived like collision between vertebrae.

Further, excessively large cage heights may result in supra physiologic interbody space restoration and potentially predispose to complications. Thus, deviations from a normative range may be included into the objective function to restore an appropriate disc height and to integrate limitations for stretching in cranial-caudal direction.

For example, a gender specific range for the anterior and posterior disc height of each level such as a L4/5 anterior disc height may be defined as 10.9 +/- 2.1 mm and a posterior disc height of 6.1+1.4 mm for men. A range of the defined mean +/-2 times standard deviation may be used as constraints for the optimization.

By constructing a parallel line to a superior endplate of an inferior vertebra which includes an endpoint of the superior endplate, an anterior respectively posterior disc height can be calculated as the distance between both lines.

Further, an anterior and posterior AP translation may be included into the objective function. For example, an ideal of 0 mm and a normative range of e.g. 2 mm may be assumed.

The optimized sagittal condition may be transferred to a 3D prediction image by applying the inverse of a 3D to 2D registration resulting in an 3D image, that is modified in respect to the sagittal aspect.

A coronal and axial alignment may be applied to the (modified) 3D prediction image. In general, the target is 0° for the Cobb angle (straight spine in AP view) and the axial rotation.

To align the AP aspect, the superior endplate of the superior vertebra and the inferior endplate of the inferior vertebrae may be projected to the AP plane. Ideally these endplates should be parallel resulting in an AP Cobb angle of 0°. To create the 3D prediction model, the superior part of the image may be rotated around the center of the superior endplate to make both endplates parallel.

The same procedure may be applied to achieve a 0° axial angle: The superior part of the 3D image may be rotated around the center of a foramen (of a vertebra) to achieve a 0° axial angle.

Based on the 3D positions of the instrumented (fused / modified) levels an automatic cage planning may be performed.

Further, following the workflow of pre-operatively generating the alignment target (e.g., determining a prediction image), a cage planning algorithm may use the target defined by the 3D prediction image as input. However, if, e.g., a surgeon cannot achieve the alignment goal, intraoperatively measured positions of the vertebrae may be used as input instead.

To determine constraints for the cage shape, an exact 3D disc space may be measured, e.g. as a convex hull between two adjacent endplates of segmented vertebrae. The objective may be to identify an ideal cage geometry that fits into the (optimized) disc space while providing contact to the endplates. Like mentioned above, the optimized 3D prediction image may be preferably used as input and just in cases where the surgeon triggers an intra-operative cage re-optimization, intra-operatively measured vertebral positions may be used as input.

A normative volume for optimal cage positions may be computed, denoted as "3D implant hull". The 3D implant hull may be a sub-volume of a disc space and may define a region where a cage should optimally be located. For different planned surgical approaches such as Transforaminal Lumbar Interbody Fusion, TLIF, or Posterior Lumbar Interbody Fusion, PLIF, different areas may be defined.

For example, a simple bounding region may be defined and an intersection of this region with a disc space may be defined as "3D implant hull" or forbidden zones may additionally be subtracted from it.

In detail, first a bounding region proportional to the geometry of the calculated disc space may be calculated. Then, an anterior boundary may be computed as the volume located anterior to the calculated disc space. Thus, the bounding region may be further limited to finally define the 3D implant hull.

Choosing the bounding region may primarily involve deciding an anterior-posterior placement of the cage, whereas the lordotic angle, the cage height and length may be derived from measurements of the 3D disc space. The lordotic angle and the cage height highly correlate with the sagittal angle and the disc height (the wedge) between the adjacent vertebrae, while the cage length may be calculated by measured proportions of the disc space. For example, in a TLIF approach, a cage may be placed maximally anterior while maximizing the length of the cage. In a lateral approach, a cage may be placed in the middle of a vertebral body while maximizing the length of the cage.

Similarly, for alternative approaches such as oblique TLIF, Anterior-To-Psoas, ATP, or PLIF, an ideal anterior-posterior placement may be defined based on the proportions of the vertebral anatomy, and the exact lordotic angle as well as the cage height may be derived from measurements of the 3D disc space (in the 3D prediction image).

After defining the 3D implant hull, a specific cage geometry (e.g., a Computer Aided Design, CAD, file) from a selected cage family may be chosen which fits best to the 3D implant hull.

In an alternative approach, the first step of computing an 3D implant hull and later selecting the best fitting cage may be omitted; and cages of a specific family might be already included in the optimization procedure. This may be implemented by one of the following options: a) possible alignments in the optimization procedure may be limited to ones that are achievable with a given family of cages; or b) a metric is included in the objective function describing the gap between the simulated alignment and the alignment that can be achieved with a given cage.

The optimization algorithm may be triggered after a lateral image has been loaded and necessary landmarks have been identified automatically or manually and confirmed by a surgeon. The resulting alignment goal may be visualized, as well as the planned cage. Further, optimal fusion levels may be visualized, e.g., as suggestion in the lateral (2D) prediction image.

A surgeon may be provided with the option to modify the wedge(s) and to re-run the optimization with the following two options:

a) Fixed Level Selection:
Surgeons may choose a specific fusion level and potentially utilize an "optimize" button to optimize the configuration for the selected level.

b) Variable Level Selection:
Surgeons may have the flexibility to select the number of fusion levels and reoptimize by using a "Calculate optimal n-level fusion" function, specifying the desired number n of levels.

Further options for changing the alignment target may be given:

- the wedge angel(s);
- the pelvic tilt; and
- the disc height(s).

For the cage, the surgeon may be able to adjust the:

- surgical approach such as traditional TLIF or PLIF;
- cage position such as the anterior-posterior placement of the cage(s); and
- cage shape such as the length.

Upon receiving a confirmation of the outlined planning, both, the cage position and the alignment target may be visualized in relation to an anatomy in a navigation view, as well as information derived from it, like the disc height as shown in Fig. 4B.

Up to this point, the described steps do not require any tracking information and may be pre-operatively computed and visualized, functioning as a standalone system 100 for cage and alignment planning (planning of a spinal cage implementation according to the first aspect described herein). Further integration of tracking information may enable intraoperative guidance for a surgeon, allowing real-time assessment of alignment goal achievement and optimal cage placement.

By tracking and visualizing a cage attached to a cage inserter (e.g., a tool configured for receiving and inserting a spinal cage) or information derived from it, a position of a navigated cage may be visually compared to a position of a planned cage. The visualization may incorporate Augmented Reality to visualize the cage position directly in-situ.

Inserting the cage and aligning the spine changes the anatomy, so it differs from a medical image used for navigation (e.g., the 3D medical image). It may thus be beneficial to display the vertebral movement during disc spreading and cage insertion in a navigation system. It may also be beneficial to show the computed lateral (2D) prediction image for the standing posture and the computed spinal parameters in real-time during the intervention.

The benefits of visualizing multiple vertebrae during the intervention may extend beyond visualization. The ability to localize vertebral movements may enable triggering of a re-planning process for an optimal cage that conforms to the current spine shape. Instead of a pre-operatively created prediction image (2D or 3D prediction images), the current position of the vertebrae may serve as input to initiate an automatic cage planning process as described herein.

The present disclosure also provides for the following examples:

1. A computer-implemented method (200) for automatically planning an implementation of one or more spinal cages, comprising:

obtaining (210) image data including a 2D medical image comprising representations of a plurality of vertebrae of a patient's body in a standing body posture;
determining (220) a 2D prediction image comprising representations of the plurality of vertebrae of the patient's body in the standing body posture, wherein determining the 2D prediction image comprises modifying the 2D medical image such that at least one spinal balance parameter derivable from the 2D prediction image fulfills a predefined criterion; and
determining (230), based on the 2D prediction image, an implementation of one or more spinal cages yielding the poses of the vertebrae as represented by the prediction image.

2. The method (200) of example 1, wherein determining (230) the implementation of the one or more spinal cages comprises transferring the poses of the vertebrae as represented by the 2D prediction image onto vertebrae represented by a 3D patient image, and determining the implementation based on the vertebrae represented by the 3D patient image having the transferred poses.

3. The method (200) of example 2, further comprising determining a registration for registering the 2D medical image with the 3D patient image, wherein the poses of the vertebrae as represented by the 2D prediction image are transferred onto vertebrae represented by the 3D patient image based on the registration of the 2D medical image with the 3D patient image.

4. The method (200) of example 2 or 3, wherein the transferred poses of vertebrae of the 3D patient image are further adjusted, in one or more planes differing from an imaging plane of the 2D medical image, before determining the implementation of the one or more spinal cages based on the vertebrae represented by the 3D patient image that have the so-adjusted poses; and, optionally, wherein
the further adjustment of the transferred poses comprises at least one of a cobb angle alignment and an axial alignment of the vertebrae.

5. The method (200) of any preceding example, wherein determining (220) the 2D prediction image comprises simulating a fusion of different vertebra levels; and, optionally, wherein

i) the fusion of different vertebra levels comprises a fusion of at least three vertebra levels; and/or
ii) the fusion is simulated based on a predefined maximum number of vertebra levels to be fused.

6. The method (200) of example 5, further comprising:

outputting a respective optimal fusion result for each number of vertebra levels to be fused;
receiving user-input selecting one of the respective optimal fusion results; and
determining the implementation of the one or more spinal cages based on the selected respective optimal fusion result.

7. The method (200) of example 5 or 6, wherein simulating the fusion of different vertebra levels comprises introducing at least one virtual wedge between vertebrae that are simulated to be fused together, for example between vertebrae represented by the 2D medical image or vertebrae represented by a or the 3D patient image; and, optionally, wherein introducing a wedge comprises splitting the obtained 2D medical image into at least two parts and moving the at least two parts relative to each other, optionally within 3 degrees of freedom.

8. The method (200) of any preceding example, wherein the at least one spinal balance parameter is indicative of at least one of a pelvic tilt, thoracic compensation, knee flexion, disc height restauration and anterior-posterior alignment.

9. The method (200) of any preceding example, further comprising obtaining one or more patient specific parameters and deriving the at least one spinal balance parameter based at least in part on the one or more patient specific parameters.

10. The method (200) of any preceding example, wherein multiple spinal balance parameters are derived from the 2D prediction image and the implementation of one or more spinal cages is determined based on a combination of at least some of the multiple spinal balance parameters, wherein, optionally, the combination comprises determining a weighted sum of the derived multiple spinal balance parameters.

11. The method (200) of example 10, wherein deriving the multiple spinal balance parameters from the 2D prediction image comprises determining multiple spinal balance parameters sets and selecting one of the sets for determining the implementation based on at least one optimization criterium.

12. The method (200) of any preceding example, wherein determining (230) the implementation of the one or more spinal cages comprises at least one of determining a position and orientation of the one or more spinal cages and determining a respective shape for each of the one or more spinal cages based at least in part on the 2D prediction image or the 3D patient image comprising the transferred poses.

13. The method (200) of example 12, further comprising selecting, based on the determined respective shapes, a spinal cage from a family of predetermined spinal cages matching the determined respective shape the best.

14. The method (200) of any preceding example, wherein determining (230) the implementation of the one or more spinal cages comprises:

determining, based at least in part on the 2D prediction image or the 3D patient image comprising the transferred poses, a region within a space between two adjusted vertebrae to place one of the one or more spinal cages in; and
determining a position for said spinal cage within the determined region based at least in part on the 2D prediction image.

15. The method (200) of examples 13 and 14, further comprising triggering display of a visualization indicating the determined spinal cage implementation together with the selected spinal cage and, optionally, indicating optimal fusion levels of the plurality of vertebrae.

16. The method (200) of example 15, wherein the visualization comprises at least one of:

i) a visualization in the 2D prediction image;
ii) a visualization in the 3D patient image comprising the transferred poses; and
iii) a visualization of the selected cage attached to a cage insertion instrument.

17. The method (200) of example 15 or 16, further comprising:

receiving user-input adjusting at least one of the visualized spinal cage implementation and the selected spinal cage; and
re-calculating the spinal cage implementation and the spinal cage shape based on the user-input; and, optionally, wherein
the user-input further indicates specific levels of vertebrae to be fused or a specific number of vertebra levels to be fused.

18. A computer program product, comprising instructions that, when executed on at least one processor, cause the at least one processor to carry out any of the methods of examples 1 to 17.

19. A planning system (100) comprising at least one processor configured to carry out the method (200) of any one of examples 1 to 17.

20. The planning system (100) of example 19, further comprising at least one of the following entities:

a medical imaging device (130) configured to generate at last one of the 2D medical image and the 3D patient image;
a user input device configured to receive the user-input;
a display device (120) configured to display the visualization.

## Claims

1. A computer-implemented method (200) for automatically planning an implementation of one or more spinal cages, comprising:

obtaining (210) image data including a 2D medical image comprising representations of a plurality of vertebrae of a patient's body in a standing body posture;
determining (220) a 2D prediction image comprising representations of the plurality of vertebrae of the patient's body in the standing body posture, wherein determining the 2D prediction image comprises modifying the 2D medical image such that at least one spinal balance parameter derivable from the 2D prediction image fulfills a predefined criterion, wherein determining (220) the 2D prediction image comprises simulating a fusion of different vertebra levels, wherein simulating the fusion of different vertebra levels comprises rotating the 2D prediction image to simulate a pelvic tilt compensation; and
determining (230), based on the 2D prediction image, an implementation of one or more spinal cages yielding the poses of the vertebrae as represented by the prediction image.

2. The method (200) of claim 1, wherein rotating the 2D prediction image to simulate a pelvic tilt compensation comprises rotating the 2D prediction image around a femur head represented in the 2D prediction image.

3. The method (200) of claim 2, wherein rotating the 2D prediction image around a femur head represented in the 2D prediction image comprises rotating the the 2D prediction image around a femur axis.

4. The method (200) of any preceding claim, wherein determining (230) the implementation of the one or more spinal cages comprises transferring the poses of the vertebrae as represented by the 2D prediction image onto vertebrae represented by a 3D patient image, and determining the implementation based on the vertebrae represented by the 3D patient image having the transferred poses.

5. The method (200) of claim 4, further comprising determining a registration for registering the 2D medical image with the 3D patient image, wherein the poses of the vertebrae as represented by the 2D prediction image are transferred onto vertebrae represented by the 3D patient image based on the registration of the 2D medical image with the 3D patient image; and/or

wherein the transferred poses of vertebrae of the 3D patient image are further adjusted, in one or more planes differing from an imaging plane of the 2D medical image, before determining the implementation of the one or more spinal cages based on the vertebrae represented by the 3D patient image that have the so-adjusted poses; and, optionally, wherein
the further adjustment of the transferred poses comprises at least one of a cobb angle alignment and an axial alignment of the vertebrae.

6. The method (200) of any preceding claim, wherein

i) the fusion of different vertebra levels comprises a fusion of at least three vertebra levels; and/or
ii) the fusion is simulated based on a predefined maximum number of vertebra levels to be fused.

7. The method (200) of claim 6, further comprising:

outputting a respective optimal fusion result for each number of vertebra levels to be fused;
receiving user-input selecting one of the respective optimal fusion results; and
determining the implementation of the one or more spinal cages based on the selected respective optimal fusion result.

8. The method of any one of claims 1 to 6, wherein simulating the fusion of different vertebra levels comprises:

starting the simulation by evaluating all 2-level fusions;
assessing higher-level fusions; and
selecting an optimal outcome from the fusions with fewer levels if an improvement resulting from adding another level falls below a predefined threshold.

9. The method (200) of any one of claims 6 to 8, wherein simulating the fusion of different vertebra levels comprises introducing at least one virtual wedge between vertebrae that are simulated to be fused together, for example between vertebrae represented by the 2D medical image or vertebrae represented by a or the 3D patient image; and, optionally, wherein
introducing a wedge comprises splitting the obtained 2D medical image into at least two parts and moving the at least two parts relative to each other, optionally within 3 degrees of freedom.

10. The method (200) of any preceding claim, wherein the at least one spinal balance parameter is indicative of at least one of a pelvic tilt, thoracic compensation, knee flexion, disc height restauration and anterior-posterior alignment; and/or
wherein the method further comprises obtaining one or more patient specific parameters and deriving the at least one spinal balance parameter based at least in part on the one or more patient specific parameters.

11. The method (200) of any preceding claim, wherein multiple spinal balance parameters are derived from the 2D prediction image and the implementation of one or more spinal cages is determined based on a combination of at least some of the multiple spinal balance parameters, wherein, optionally:

i) the combination comprises determining a weighted sum of the derived multiple spinal balance parameters; and/or
ii) deriving the multiple spinal balance parameters from the 2D prediction image comprises determining multiple spinal balance parameters sets and selecting one of the sets for determining the implementation based on at least one optimization criterion.

12. The method (200) of any preceding claim, wherein determining (230) the implementation of the one or more spinal cages comprises at least one of determining a position and orientation of the one or more spinal cages and determining a respective shape for each of the one or more spinal cages based at least in part on the 2D prediction image or the 3D patient image comprising the transferred poses.

13. The method (200) of claim 12, further comprising selecting, based on the determined respective shapes, a spinal cage from a family of predetermined spinal cages matching the determined respective shape the best.

14. The method (200) of any preceding claim, wherein determining (230) the implementation of the one or more spinal cages comprises:

determining, based at least in part on the 2D prediction image or the 3D patient image comprising the transferred poses, a region within a space between two adjusted vertebrae to place one of the one or more spinal cages in; and
determining a position for said spinal cage within the determined region based at least in part on the 2D prediction image.

15. The method (200) of claims 13 and 14, further comprising triggering display of a visualization indicating the determined spinal cage implementation together with the selected spinal cage and, optionally, indicating optimal fusion levels of the plurality of vertebrae.

16. The method (200) of claim 15, wherein the visualization comprises at least one of:

i) a visualization in the 2D prediction image;
ii) a visualization in the 3D patient image comprising the transferred poses; and
iii) a visualization of the selected cage attached to a cage insertion instrument.

17. The method (200) of claim 15 or 16, further comprising:

receiving user-input adjusting at least one of the visualized spinal cage implementation and the selected spinal cage; and
re-calculating the spinal cage implementation and the spinal cage shape based on the user-input; and, optionally, wherein
the user-input further indicates specific levels of vertebrae to be fused or a specific number of vertebra levels to be fused.

18. A computer program product, comprising instructions that, when executed on at least one processor, cause the at least one processor to carry out any of the methods of claims 1 to 17.

19. A planning system (100) comprising at least one processor configured to carry out the method (200) of any one of claims 1 to 17.

20. The planning system (100) of claim 19, further comprising at least one of the following entities:

a medical imaging device (130) configured to generate at last one of the 2D medical image and the 3D patient image;
a user input device configured to receive the user-input;

a display device (120) configured to display the visualization.

Fig. 1

**200**

**210** — Obtain image data including a 2D medical image comprising representations of a plurality of vertebrae of a patient's body in a standing body posture

**220** — Determine a 2D prediction image comprising representations of the plurality of vertebrae of the patient's body in the standing body posture, wherein determining the 2D prediction image comprises modifying the 2D medical image such that at least one spinal balance parameter derivable from the 2D prediction image fulfills a predefined criterion

**230** — Determine, based on the 2D prediction image, an implementation of one or more spinal cages yielding the poses of the vertebrae as represented by the prediction image

Fig. 2

Fig. 3A

Fig. 3B

EP 4 755 337 A2

Fig. 4A

Fig. 4B